# EUROPEAN PATENT SPECIFICATION

(11) **EP 2 214 551 B1**
(45) Date of publication and mention of the grant of the patent: **12.10.2011**
(21) Application number: 08843880.9
(22) Date of filing: 03.11.2008
(51) Int. Cl.: A61B 5/00

(54) **MEASUREMENT OF OXYGEN SATURATION OF BLOOD HAEMOGLOBIN**
MESSUNG DER SAUERSTOFFSÄTTIGUNG VON BLUTHÄMOGLOBIN
MESURE DE LA SATURATION EN OXYGENE DE L'HEMOGLOBINE SANGUINE

(30) Priority: 02.11.2007 GB 0721575
(43) Date of publication of application: 11.08.2010
(73) Proprietor: Intelesens Limited, Belfast BT3 9LE (GB)
(72) Inventor: McLAUGHLIN, James, Andrew, Belfast BT7 2GH (GB); ANDERSON, John, McCune, Hollywood B18 0NG (GB)
(86) International application number: PCT/GB2008/003708
(87) International publication number: WO 2009/056859

(56) References cited:
- EP-A- 1 946 697
- WO-A-96/16591
- WO-A-99/47039
- GB-A- 2 425 181
- US-A1- 2002 124 295
- US-A1- 2007 142 715

## Description

This invention relates to the measurement of oxygen saturation of blood haemoglobin.

The oxygen saturation of haemoglobin in blood is an important indicator of the health of a subject. For example, measurements of oxygen saturation can detect hypoxia before the subject becomes cyanosed. Measurement of oxygen saturation of blood haemoglobin is therefore routinely carried out for subjects receiving medical care in hospitals, and for monitoring the health of subjects in the home.

Traditionally, oximeters are used to make measurements of oxygen saturation of blood haemoglobin at peripheral sites of the subject, such as a finger, ear or toe. Thus saturation of peripheral oxygen, commonly referred to as SpO₂ is measured. In use, an oximeter is attached to a peripheral site of the subject, in proximity to an artery, and senses the oxygen saturation of arterial blood. The oximeter comprises two radiation sources and a radiation detector. Commonly, the radiation sources are positioned on a first side of the peripheral site, e.g. on a first side of a finger, and the radiation detector is arranged on a second, opposite, sidle of the peripheral site. This is referred to as a transmission oximeter. Radiation from the sources is transmitted from the sources into the peripheral site. Some of the radiation is absorbed by the peripheral site, and particularly the blood in the artery of the site, and some of the radiation passes through the peripheral site. At least some of this transmitted radiation is detected by the detector. The radiation sources, usually LEDs, produce radiation at different wavelengths, the first source in the red part of the electromagnetic spectrum, and the second source in the infra red (IR) part of the electromagnetic spectrum. The level of absorption of red and IR radiation in blood, depends on the oxygenation level of haemoglobin in the blood. Further, for blood having a particular haemoglobin oxygenation level, the red and IR radiation will be absorbed by different amounts. By detecting radiation that is transmitted through the arterial blood, it is possible to calculate the absorption of the red and IR radiation and compute the percentage of haemoglobin in the arterial blood which is saturated with oxygen. This is usually expressed as a percentage of total saturation. The blood flow through the artery will be pulsatile in form. The oximeter is designed to detect radiation transmitted through blood in the artery, by being configured to detect pulsatile transmitted radiation. The oximeter is able to distinguish the pulsatile signals from other more static signals, e.g. signals transmitted through tissue or veins.

The oximeter is able to measure the heart rate of the subject, and a!so to produce an indication of the quality of blood flow through the artery.

As measurement of oxygen saturation of haemoglobin in blood finds widespread application, improvements in measurement systems and techniques are constantly being sought.

WO 99/47039 discloses an infrared oximeter, which can be placed more freely and at more locations on the body than normal oximeter probes. It comprises a light transmitter and receiver for measuring saturation of haemoglobin in the blood and a fastener that can be placed around the chest of a subject and which applies pressure to the oximeter.

US 2002/0124295 discloses a carrier for a biophysical sensor in a clothing apparatus. The biophysical sensor can measure saturation of haemoglobin in the blood and can be held in place over the chest of a subject by a band of stretch fabric.

WO 96/16591 discloses a device for monitoring vital functions such as saturation of haemoglobin in the blood. The device comprises an oximeter having a light source and receiver and a strap to attach the oximeter to the chest of a subject and apply pressure to the oximeter.

jGB 2425181 discloses a wearable physiological monitoring device which comprises an oximeter for measuring saturation of haemoglobin in the blood and which can be attached to the chest of a subject by a strap-on harness which applies pressure to the oximeter.

US 2007/0142715 discloses a chest strap for measuring vital signs such as saturation of haemoglobin in the blood using transmit and receive LEDS of an oximeter.

According to a first aspect of the invention there is provided a chest-based oximeter for measuring oxygen saturation of haemoglobin in blood of the chest of a subject, comprising at least one radiation source adapted to emit radiation onto the chest, at least one radiation detector adapted to detect radiation reflected from the chest,
a pressure device adapted to apply pressure to the oximeter to connect the oximeter to the chest, and
a hydrogel interface situated between the at least one radiation source and the at least one radiation detector and the chest of the subjet.

It has been found that the signals representing radiation reflected from the chest are weaker than signals representing radiation transmitted through the finger. Nevertheless, the chest signals are measurable, are sufficiently strong to measure accurately oxygen saturation values, oxygen perfusion trends and accurate heart rate values, and are sufficiently repeatability for good quality measurements.

The pressure device may be profiled to press onto the chest of the subject to apply pressure to the oximeter towards the chest, to connect the oximeter to the chest. The pressure device may comprise a suction device. The pressure device may comprise a biasing device, for example, a spring. The pressure device may comprise a finger push device. The pressure device may comprise a belt.

The pressure device may apply a pressure in the range of approximately 1 Pascal to approximately 100000 Pascal. The pressure device may be provided with a pressure sensitive adhesive.

The pressure device may optically couple the radiation from the radiation source to the chest of the subject. The pressure device may optically couple the radiation reflected from the chest of the subject to the radiation detector.

It has been found that the amplitude of detected radiation significantly increases when a pressure is applied to the oximeter. The amplitude of the detected radiation is indicative of the quality of the measure of the oxygen saturation of haemoglobin in blood that is obtained by the oximeter. Accurate measurement of oxygen saturation by oximeters can only be achieved when an adequate pulse is present at the measurement site, i.e. the chest. Applying pressure to the oximeter will increase the strength of the measurement of the pulse and therefore oxygen saturation will be more accurately measured by the oximeter. The pressure to be applied by the pressure device may be determined by applying a range of pressure from zero pressure to heavy pressure to the oximeter, and measuring the radiation reflected from the chest. It has been found that as the pressure is gradually increased, the peak to peak values of measured signals stays approximately constant until a pressure threshold is reached, then the peak to peak values increases as the pressure continues to increase, until a cut-off pressure is reached at which the peak to peak values fall to an unmeasurable size where the quality of the signals has deteriorated significantly. The pressure range between the pressure threshold and the pressure cut-off is the optimum pressure range to use.

The chest-based oximeter may comprise an optical coupling element. This may be positioned in the oximeter to enhance coupling of radiation between the radiation source and the radiation detector and the chest of the subject.

The at least one radiation source and the at least one radiation detector may be mounted in the chest-based oximeter such that they are spaced apart by a distance in the range of approximately 0.5mm to approximately 2cm. The at least one radiation source and the at least one radiation detector may be mounted in the chest-based oximeter such that, in use, they are positioned in the range of approximately 1 cm to approximately 20cm above the chest of the subject.

The at least one radiation source may emit radiation having one or more infra red peak wavelengths. The chest-based oximeter may further comprise a second radiation source which emits radiation having one or more infra red peak wavelengths. The at least one radiation source may emit radiation having at least a first infra red peak wavelength, and the second radiation source may emit radiation having at least a second, different, infra red peak wavelength. The or each infra red peak wavelength is in the range of 600nm to 1500nm, for example 780nm, 810nm, 820nm, 830nm, 840nm, 850nm, 870nm, 880nm, 890nm, 910nm, 940nm, 970nm, 1050nm, 1070nm, 1200nm, 1300nm, 1350nm, 1450nm, 1550nm.

Infra red radiation has a wavelength range which sensitive to the measurement of oxygen saturation in blood. Using one or more radiation sources which emit radiation having infra red wavelengths therefore optimises the detection ability of the oximeter. Effects due to skin, tissue path, etc. can be filtered or monitored and subtracted in order to measure the oxygen saturation and also the heart rate of the subject.

The at least one radiation source may emit radiation having one or more visible peak wavelengths. The chest-based oximeter may further comprise a second radiation source which emits radiation having one or more visible peak wavelengths.

The at least one radiation source may emit radiation having one or more visible peak wavelengths, and the oximeter may further comprise a second radiation source which emits radiation having one or more infra red peak wavelengths.

The radiation source or radiation sources may comprise an LED or LEDs. The radiation source or radiation sources may comprise a solid state laser or solid state lasers. When the oximeter comprises two or more radiation sources, the sources may comprise LEDS, or solid state lasers, or a combination of LEDs and solid state lasers.

The chest-based oximeter may comprise one or more devices to, for example, process or amplify the radiation detected by the radiation detector. The chest-based oximeter may comprise a processor, which may receive one or more signals representing radiation detected by the radiation detector, and may use the signal or signals to provide a measure of the oxygen saturation of haemoglobin in blood of the chest. The chest-based oximeter may comprise a transmitter. The transmitter may transmit one or more signals representing the measure of the oxygen saturation of haemoglobin in blood of the chest to a remote receiver. The transmitter may relay changes in oxygen saturation in blood of the chest of the subject to, for example, a clinician even when the subject is at home. The chest-based oximeter may comprise a receiver. The receiver may receive instructions which may be used to control the operation of the chest-based oximeter.

Alternatively, the transmitter may transmit signals representing the radiation detected by the detector to a remote receiver, which comprises a processor which uses the signal or signals to provide a measure of the oxygen saturation of haemoglobin in blood of the chest.

The transmitter may wirelessly transmit the one or more signals to the remote receiver. This means that the chest-based oximeter does not require leads to connect to the remote receiver, which may otherwise impede movement of the subject.

The chest-based oximeter comprises a hydrogel interface. The hydrogel interface may, in use, be attached to the chest of the subject. The hydrogel interface may be placed in contact with skin of the chest. The hydrogel interface may comprise adhesive, which is used to attach it to the skin. The adhesive may be a pressure-sensitive adhesive. The hydrogel interface may have substantially similar visco-elastic properties as skin. The hydrogel interface may have a Young's modulus substantially similar to that of skin. The hydrogel interface may be flexible. The hydrogel interface may be flexible to allow it to flex with movement of the subject. The hydrogel interface may be flexible to allow it to flex with movement of the subject, such that it remains attached to the chest. The hydrogel interface may be flexible to allow it to flex with movement of the subject, such that radiation from the radiation source is emitted substantially perpendicular onto the measurement site of the subject. The hydrogel interface may be flexible to allow it to flex with movement of the subject, such that motion-induced artefacts in the reflected radiation detected by the detector are reduced. The hydrogel interface may have substantially similar electrical properties as skin. This will give overall similar ionic content and therefore no potential gradients. The hydrogel interface may act as a second skin for the measurement site.

The hydrogel interface is situated between the radiation source and radiation detector and the chest of the subject. The hydrogel interface may cover the radiation source. The hydrogel interface may cover the radiation detector. The radiation source may emit radiation through the hydrogel interface onto the chest. The hydrogel interface may diffuse the radiation emitted from the radiation source. The diffusion of the radiation may average angles of penetration of the radiation into the chest. The diffusion may be, for example, from an approximately 1mm² source to an area of approximately 1cm². The hydrogel interface may provide coupling of the radiation from the source to the chest. The radiation detector may detect radiation reflected from the chest which passes through the hydrogel interface. The hydrogel interface may diffuse the radiation reflected from the chest. The diffusion of the radiation may average angles of reflection of the radiation from the chest. The diffusion may be, for example, from an approximately 1mm² source to an area of approximately 1cm². The hydrogel interface may provide coupling of the radiation from the chest to the radiation detector. The diffusion allows for improved averaging of the absorption of the radiation, and thus improved accuracy of the oximeter. The coupling improves the detection of the reflected radiation against background noise/artefact. This gives a cleaner and more sensitive analysis leading to higher accuracy. The optical coupling stops stray light from interfering with the detected radiation.

The hydrogel interface may be shaped to fit the chest of the subject. The hydrogel interface may comprise a film. The hydrogel interface may comprise a ball. The hydrogel interface may have a thickness in the range of approximately 0.5mm to approximately 1cm. The hydrogel interface may have an area of approximately 1cm². The hydrogel interface may be up to approximately 85% water based. The hydrogel interface is preferably biocompatible. It will then have a low toxicity and sensitisation effects on the subject.

It has been found that when the chest-based oximeter is provided with a hydrogel interface, the signals produced by the radiation detector have waveforms which can be sharper and more consistent, than those produced when no hydrogel interface is used. When no hydrogel interface is used, the signals produced by the radiation detector are stronger than those produced when a hydrogel interface is used, but the signals may be more prone to influence by slight movements between the oximeter and the skin of the subject and between the skin and underlying bone.

The chest-based oximeter may have a low profile. This will make it easier to wear than conventional oximeters used on a finger, ear or toe.

The chest-based oximeter may, in use, be attached to a region of the chest above the notch sternum. Positioning of the chest-based oximeter above the notch region of the chest of the subject will ensure high coupling of the oximeter with the main heart blood paths. Positioning of the chest-based oximeter above the notch region of the chest of the subject avoids hysteresis or time delays in signals detected by the oximeter. Positioning of the chest-based oximeter above the notch region of the chest of the subject also makes the oximeter easy for the subject to wear.

The chest-based oximeter may be used to measure a wide range of oxygen saturation values. This may be tested by the subject carrying out a controlled breathing exercise, which manipulates the subject's blood oxygen saturation values, through a wide range of saturation values. It has been found that decreases in oxygen saturation values were indicated earlier at the chest than at the finger, that measurements of oxygen saturation taken at the chest fell at a faster rate than measurements of oxygen saturation taken at the finger, and that chest oxygen saturation values fell to a lower level than finger oxygen saturation values.

The chest-based oximeter may also measure carbon monoxide saturation in blood of the chest of the subject.

The chest-based oximeter may form part of a system which measures one or more vital signs of the subject. The vital signs may comprise any of heart rate, ECG, respiration rate, temperature. The system may comprise the V-patch vital signs measurement system of Sensor Technology & Devices.

According to a second aspect of the invention there is provided a method of measuring oxygen saturation of haemoglobin in blood of the chest of a subject, comprising
attaching an oximeters to the chest using a pressure device of the oximeter adopted to apply pressure thereto to connect the oximeter to the chest. The oximeter comprising a hydrogel interface, operating at least one radiation source of the oximeter to emit radiation onto the chest,
operating at least one radiation detector of the oximeter to detect radiation reflected from the chest, and
using the radiation detected by the detector to measure the oxygen saturation of haemoglobin in blood of the chest.

Embodiments of the invention will now be described by way of example only, with reference to the accompanying drawings, in which:
Figure 1 is a schematic representation of a first embodiment of an chest-based oximeter according to the invention, shown positioned on a subject's chest;
Figure 2 is a schematic representation of the chest-based oximeter of Figure 1, and
Figure 3 is a schematic representation of a second embodiment of a chest-based oximeter according to the invention.

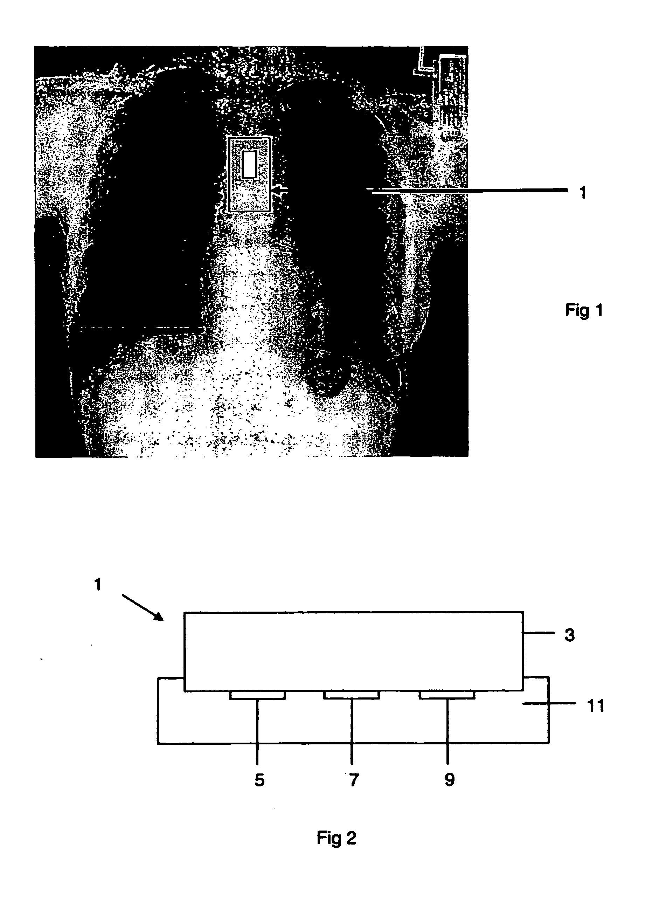

Figure 1 shows a first embodiment of a chest-based oximeter according to the invention, placed on a subject's chest. The oximeter is positioned on a region of the chest above the notch sternum, as illustrated, and measures oxygen saturation of haemoglobin in blood of the region of the chest above the notch sternum.

Figure 2 shows a chest-based oximeter 1 in more detail not in accordance with the invention. The oximeter 1 comprises a housing 3, two radiation sources 5, 7, a radiation detector 9 and a pressure device 11. The housing comprises a flexible substrate. The radiation sources 5, 7 and the radiation detector 9 are each mounted on a surface of the housing 3, as shown, in a reflectance measurement arrangement. The sources and the detector are spaced apart by a distance in the range of approximately 0.5mm to approximately 2cm. The radiation sources 5, 7 comprise LEDs. The radiation source 5 emits radiation having a visible peak wavelength in the red region of the visible spectrum. The radiation source 7 emits radiation having an infra red peak wavelength of 920nm or 1300nm. The radiation source 7 comprises an IR LED, such as those supplied by Roithner Lasertechnik. The radiation detector 9 comprises a photodiode, such as the TSL220 photodiode supplied by Texas Instruments.

As described, the chest-based oximeter 1 comprises a conventional arrangement of radiation sources in terms of number and wavelength. It will be appreciated that the oximeter 1 could comprise a different number of radiation sources with different wavelengths, for example a first radiation source which emits radiation having an infra red peak wavelength of 900nm and a second radiation source which emits radiation having an infra red peak wavelength of 1300nm.

The pressure device 11 comprises a stretchable foam material, which has a Young's modulus which is lower than that of the skin of the chest of the subject. The pressure device 11 is attached at a first side thereof to the surface of the housing 3 on which the radiation sources and detector are mounted. The pressure device 11 as shown extends along the surface of the housing 3 and around the sides of the housing 3. It will be appreciated that other shapes of pressure device 11 may be used, for example, the pressure device may only extend along part of the surface of the housing 3. In use, the pressure device 11 is applied at a second side thereof to skin of the region of the chest above the notch sternum of the subject. As the Young's modulus of the skin and the Young's modulus of the pressure device material are different, this will cause the pressure device 11 to stress and apply a pressure on the oximeter towards the skin, and connect the oximeter to the chest. The pressure device 11 may apply a pressure in the range of approximately 1 Pascal to approximately 100000 Pascal on the oximeter towards the chest of the subject.

The pressure device 11 is of a thickness to position the radiation sources 5, 7 and the radiation detector 9 between approximately 1cm and approximately 20 cm above the notch region of the chest. The pressure device 11 is shaped to optically couple the radiation from the radiation sources 5, 7 to the notch region of the chest, and to optically couple the radiation reflected from the notch region of the chest to the radiation detector 9.

The chest-based oximeter 1 further comprises a power supply, a controller, a processor, a receiver, a transmitter and electronic circuitry, all located within the housing 3. The electronic circuitry connects the radiation sources 5, 7 and the radiation detector 9 to the power supply, for supply of power thereto. It will be appreciated that, alternatively, a power supply could be provided external to the oximeter 1. The electronic circuitry connects the radiation sources 5, 7 to the controller, which acts to control the operation of the sources. The electronic circuitry connects the radiation detector 9 to the processor. The processor receives signals from the radiation detector 9, and uses the signals to provide a measure of the oxygen saturation. The processor may pass measurement of the oxygen saturation to the transmitter, for transmission to a device external to the oximeter. It will appreciated that the chest-based oximeter 1 may be connected to a processor which is situated external to the oximeter. The oximeter may then transmit signals from the radiation detector 9 to a receiver of the external processor. The controller of the oximeter 1 may receive control signals via the receiver from a source external to the oximeter, to control operation of the oximeter. For example, the control signals may be generated by a physician of the subject. The transmitter and receiver of the oximeter 1 may be connected by wires or, preferably, wirelessly connected to devices external to the oximeter.

The entire chest-based oximeter 1 has a low profile. This will make it easier for the subject to wear than conventional oximeters used on a finger, ear or toe.

In use, the chest-based oximeter 1 is attached to the region of the chest above the notch sternum of the subject using the pressure device 11, and measures oxygen saturation of haemoglobin in blood of the notch region of the chest, as follows. The controller receives a signal via the receiver causing it to activate the radiation sources 5, 7. These emit red and IR radiation onto the notch region of the chest. The radiation from the sources is transmitted into the notch region, and some of the radiation is absorbed by the notch region of the chest, and particularly haemoglobin in blood in arteries of the chest region. Some of the radiation from the sources is reflected from the notch region of the chest, and particularly haemoglobin in the blood in the arteries. The level of absorption of red and IR radiation in blood, depends on the oxygenation level of haemoglobin in the blood, and, for blood having a particular haemoglobin oxygenation level, the red and IR radiation will be absorbed by different amounts. The detector 9 detects red and IR radiation reflected from the notch region. The detector 9 produces signal representative of the reflected radiation, and passes these signals to the processor. The processor uses the signals in an analysis algorithm and calculates the absorption of the red and IR radiation, and computes a measure of the oxygen saturation of haemoglobin in blood of the arteries of the notch sternum region of the chest. This is usually expressed as a percentage of total saturation. The blood flow through the arteries will be pulsatile in form. The oximeter 1 is designed to detect radiation reflected from blood in the arteries, by being configured to detect pulsatile reflected radiation. The oximeter 1 is able to distinguish the pulsatile signals from other more static signals, e.g. signals transmitted through tissue or veins. The oximeter 1 is also able to measure the heart rate of the subject, and to produce an indication of the quality of blood flow through the arteries.

Positioning of the oximeter 1 above the notch region of the chest of the subject ensures high coupling of the oximeter 1 with the main heart blood arteries. Positioning of the oximeter 1 above the notch region of the chest also avoids hysteresis in signals detected by the oximeter 1, and makes the oximeter easy for the subject to wear.

As stated earlier, it has been found that the signals representing radiation reflected from the chest are weaker than signals representing radiation transmitted through the finger. Nevertheless, the chest signals are measurable, are sufficiently strong to measure accurately oxygen saturation values and accurate heart rate values and are sufficiently repeatability for good quality measurements.

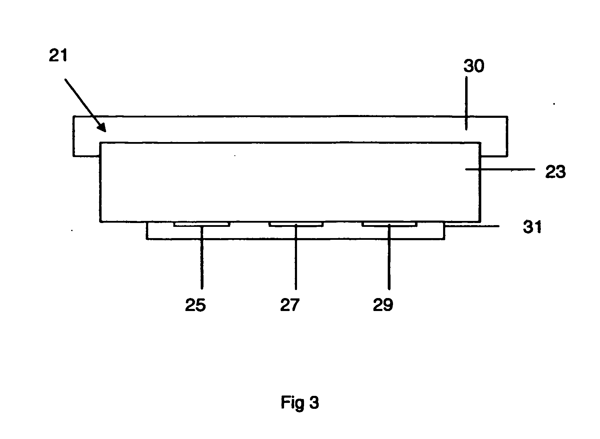

Figure 3 shows an embodiment of a chest-based oximeter 21 , used to measure oxygen saturation of haemoglobin in blood of a subject. The oximeter 21 comprises a housing 23, two radiation sources 25, 27, a radiation detector 29, a pressure device 30 and a hydrogel interface 31. The housing comprises a flexible substrate. The radiation sources 25, 27 and the radiation detector 29 are each mounted on a surface of the housing 23, as shown, in a reflectance measurement arrangement. The sources and the detector are spaced apart by a distance in the range of approximately 0.5mm to approximately 2cm. The radiation sources 25, 27 comprise LEDs. The radiation source 25 emits radiation having a visible peak wavelength in the red region of the visible spectrum. The radiation source 27 emits radiation having an infra red peak wavelength. As described, the chest-based oximeter 21 again comprises a conventional arrangement of radiation sources in terms of number and wavelength. It will be appreciated that the oximeter 21 could comprise a different number of radiation sources with different wavelengths, for example one or two radiation sources which emit radiation having one or more infra red peak wavelengths.

The pressure device 30 may comprise any of a finger push device, a belt, a biasing device, for example, a spring. The pressure device 30 is attached at a first side thereof to the surface of the housing 23 on which the radiation sources and detector are not mounted. The pressure device 30 as shown extends along the surface of the housing 23 and around the sides of the housing 23. It will be appreciated that other shapes of pressure device 30 may be used, for example, the pressure device may only extend along part of the surface of the housing. In use, the oximeter 21 is applied to the region of the chest above the notch sternum of the subject. The pressure device 30 is activated and applies a pressure on the oximeter 21 towards the chest, and connects the oximeter 21 to the chest. The pressure device 11 may apply a pressure in the range of approximately 1 Pascal to approximately 100000 Pascal on the oximeter towards the chest of the subject.

The hydrogel interface 31 is shaped to fit the chest of the subject. The hydrogel interface 31 comprises a film, having a thickness in the range of approximately 0.5mm to approximately 1 cm. The hydrogel interface 31 is biocompatible, to reduce toxicity and sensitisation effects on the subject.

The hydrogel interface 31 is attached at a first side thereof to the surface of the housing 23 on which the radiation sources and detector are mounted. The hydrogel interface 31 is attached at a second side thereof to the chest of the subject. The hydrogel interface 31 is placed in contact with skin of the measurement site, and comprises adhesive, which is used to attach it to the skin. The adhesive is preferably pressure-sensitive. The hydrogel interface 31 has substantially similar visco-elastic properties as skin, and a Youngs modulus substantially similar to that of skin. The hydrogel interface 31 is flexible, to allow it to flex with movement of the subject, such that it remains attached to the chest and radiation from the radiation sources is emitted substantially perpendicular onto the measurement site. Such flexibility of the hydrogel interface 31 will reduced motion-induced artefacts in the reflected radiation detected by the detector 29. The hydrogel interface 31 preferably also has substantially similar electrical properties as skin. In effect, the hydrogel interface 31 acts as a second skin for the chest of the subject.

The hydrogel interface 31 is situated between the radiation sources 25, 27 and the radiation detector 29, and the chest of the subject, as shown. The hydrogel interface 31 thus covers the radiation sources 25, 27, which emit radiation through the hydrogel interface 31 onto the chest. The hydrogel interface 31 diffuses the radiation emitted from the radiation sources, to average angles of penetration of the radiation into the chest. The hydrogel interface 31 also provides coupling of the radiation from the sources 25, 27 to the chest. The hydrogel interface 31 also covers the radiation detector 29, which detects radiation reflected from the chest which passes through the hydrogel interface 31. The hydrogel interface 31 diffuses the radiation reflected from the chest, to average angles of reflection of the radiation from the chest. The hydrogel interface 31 also provides coupling of the radiation from the chest to the radiation detector 29.

The oximeter 21 further comprises a power supply, a controller, a processor, a receiver, a transmitter and electronic circuitry, all located within the housing 23. The electronic circuitry connects the radiation sources 25, 27 and the radiation detector 29 to the power supply, for supply of power thereto. It will be appreciated that, alternatively, a power supply could be provided external to the oximeter 21. The electronic circuitry connects the radiation sources 25, 27 to the controller, which acts to control the operation of the sources. The electronic circuitry connects the radiation detector 29 to the processor. The processor receives signals from the radiation detector 29, and uses the signals to provide a measure of the oxygen saturation. The processor may pass measurement of the oxygen saturation to the transmitter, for transmission to a device external to the oximeter. It will appreciated that the chest-based oximeter may be connected to a processor which is situated external to the oximeter 21. The oximeter may then transmit signals from the radiation detector 29 to a receiver of the external processor. The controller of the oximeter 21 may receive control signals via the receiver from a source external to the oximeter, to control operation of the oximeter. For example, the control signals may be generated by a physician of the subject. The transmitter and receiver of the oximeter 21 may be connected by wires or, preferably, wirelessly connected to devices external to the oximeter.

In use, the oximeter 21 is attached to the chest of the subject via the hydrogel interface 31, and the pressure device 30 activated to apply pressure on the oximeter towards the chest. The region of the chest of the subject may be above the notch sternum of the chest. The controller receives a signal via the receiver causing it to activate the radiation sources 25, 27. These emit red and IR radiation onto the measurement site. The radiation from the sources is transmitted into the chest, and some of the radiation is absorbed by the chest, and particularly haemoglobin in blood in arteries of the chest. Some of the radiation from the sources is reflected from the chest, and particularly haemoglobin in the blood in the arteries. The level of absorption of red and IR radiation in blood, depends on the oxygenation level of haemoglobin in the blood, and, for blood having a particular haemoglobin oxygenation level, the red and IR radiation will be absorbed by different amounts. The detector 29 detects red and IR radiation reflected from the chest. The detector 29 produces signal representative of the reflected radiation, and passes these signals to the processor. The processor uses the signals in an analysis algorithm and calculates the absorption of the red and IR radiation, and computes a measure of the oxygen saturation of haemoglobin in blood of the arteries of the chest. This is usually expressed as a percentage of total saturation. The blood flow through the arteries will be pulsatile in form. The oximeter 21 is designed to detect radiation reflected from blood in the arteries, by being configured to detect pulsatile reflected radiation. The oximeter 21 is able to distinguish the pulsatile signals from other more static signals, e.g. signals transmitted through tissue or veins. The oximeter 21 is also able to measure the heart rate of the subject, and to produce an indication of the quality of blood flow through the arteries.

It has been found that when the chest-based oximeter 21 is provided with a hydrogel interface 31, the signals produced by the radiation detector 29 have waveforms which are sharper and more consistent, than those produced when no hydrogel interface is used. When no hydrogel interface is used, the signals produced by the radiation detector are stronger than those produced when a hydrogel interface is used, but the signals are more prone to influence by slight movements between the oximeter and the skin of the subject and between the skin and underlying bone.

## Claims

1. A chest-based oximeter (21) for measuring oxygen saturation of haemoglobin in blood of the chest of a subject, comprising
at least one radiation source (25, 27) adapted to emit radiation onto the chest,
at least one radiation detector (29) adapted to detect radiation reflected from the chest,
a pressure device (30) adapted to apply pressure to the oximeter to connect the oximeter to the chest, and **characterized by**:
a hydrogel interface (31) situated between the at least one radiation source (25, 27) and the at least one radiation detector (29) and, in use, the chest of the subject.

2. A chest-based oximeter (21) according to claim 1 in which, in use, the at least one radiation source (25, 27) emits radiation through the hydrogel interface (31) onto the chest, and the hydrogel interface (31) diffuses the radiation emitted from the radiation source (25, 27).

3. A chest-based oximeter (21) according to claim 1 or claim 2 in which, in use, the at least one radiation source (25, 27) emits radiation through the hydrogel interface (31) onto the chest, and the hydrogel interface (31) provides coupling of the radiation from the source (25, 27) to the chest

4. A chest-based oximeter (21) according to any preceding claim in which, in use, the at least one radiation detector (29) detects radiation reflected from the chest which passes through the hydrogel interface (31), and the hydrogel interface (31) diffuses the radiation reflected from the chest.

5. A chest-based oximeter (21) according to any preceding claim in which, in use, the at least one radiation detector (29) detects radiation reflected from the chest which passes through the hydrogel interface (31), and the hydrogel interface (31) provides coupling of the radiation from the chest to the radiation detector (29).

6. A chest-based oximeter (21) according to any preceding claim in which the pressure device (30) comprises any of a biasing device, a finger push device, a belt

7. A chest-based oximeter (21) according to any preceding claim in which the at least one radiation source (27) emits radiation having one or more infra red peak wavelengths.

8. A chest-based oximeter (21) according to claim 7 which further comprises a second radiation source which emits radiation having one or more infra red peak wavelengths.

9. A chest-based oximeter (21) according to claim 8 in which the at least one radiation source (27) emits radiation having at least a first infra red peak wavelength, and the second radiation source emits radiation having at least a second, different, infra red peak wavelength.

10. A chest-based oximeter (21) according to any of claims 7 to 9 in which the or each infra red peak wavelength is the range of 600nm to 1500nm, for example, 780nm, 810nm, 820nm, 830nm, 840nm, 850nm, 870nm, 880nm. 890nm, 910nm, 940nm, 970nm, 1050nm, 1070nm, 1200nm, 1300nm, 1350nm, 1450nm, 1550nm.

11. A chest-based oximeter (21) according to any of claims 1 to 6 in which the at least one radiation source (25) emits radiation having one or more peak wavelengths in the visible spectrum and the oximeter (21) further comprises a second radiation source which emits radiation having one or more peak wavelengths in the visible spectrum.

12. A chest-based oximeter (21) according to any of claims 1 to 6 in which the at least one radiation source (25) emits radiation having one or more peak wavelengths in the visible spectrum, and the oximeter further comprises a second radiation source which emits radiation having one or more infra red peak wavelengths.

13. A chest-based oximeter (21) according to any preceding claim which has a low profile.

14. A chest-based oximeter (21) according to any preceding claim which forms part of a system which measures one or more vital signs of the subject, such as any of heart rate, ECG, respiration rate, temperature.

15. A method of measuring oxygen saturation of haemoglobin in blood of the chest of a subject comprising
attaching an oximeter (21) to the chest using a pressure device (30) of the oximeter adapted to apply pressure thereto to connect the oximeter to the chest, the oximeter comprising a hydrogel interface (31),
operating at least one radiation source (25, 27) of the oximeter to emit radiation onto the chest,
operating at least one radiation detector (29) of the oximeter to detect radiation reflected from the chest, and
using the radiation detected by the detector (29) to measure the oxygen saturation of haemoglobin in blood of the chest

## Patentansprüche

1. Auf dem Brustkorb aufsitzendes Oximeter (21) zur Messung der Sauerstoffsättigung des Hämoglobins im Blut des Brustkorbs eines Patienten, umfassend
mindestens eine Strahlungsquelle (25, 27), die dazu eingerichtet ist, Strahlung auf den Brustkorb auszusenden,
mindestens einen Strahlungsdetektor (29), der dazu eingerichtet ist, die vom Brustkorb reflektierte Strahlung zu erfassen,
eine Druckvorrichtung (30), die dazu eingerichtet ist, Druck auf das Oximeter auszuüben, um das Oximeter mit dem Brustkorb zu verbinden, und **dadurch gekennzeichnet, dass**
sich eine Hydrogel-Schnittstelle (31) zwischen der mindestens einen Strahlungsquelle (25, 27) und dem mindestens einen Strahlungsdetektor (29) und - bei der Anwendung - dem Brustkorb des Patienten befindet.

2. Auf dem Brustkorb aufsitzendes Oximeter (21) gemäß Anspruch 1, bei dem - bei der Anwendung - die mindestens eine Strahlungsquelle (25, 27) durch die Hydrogel-Schnittstelle (31) Strahlung auf den Brustkorb aussendet und die Hydrogel-Schnittstelle (31) die von der Strahlungsquelle (25, 27) ausgesendete Strahlung zerstreut.

3. Auf dem Brustkorb aufsitzendes Oximeter (21) gemäß Anspruch 1 oder Anspruch 2, bei dem - bei der Anwendung - die mindestens eine Strahlungsquelle (25, 27) durch die Hydrogel-Schnittstelle (31) Strahlung auf den Brustkorb aussendet und die Hydrogel-Schnittstelle (31) für die Kopplung der Strahlung von der Quelle (25, 27) zum Brustkorb sorgt.

4. Auf dem Brustkorb aufsitzendes Oximeter (21) gemäß einem der vorherigen Ansprüche, bei dem - bei der Anwendung - der mindestens eine Strahlungsdetektor (29) die vom Brustkorb reflektierte Strahlung erfasst, welche die Hydrogel-Schnittstelle (31) durchdringt, und die Hydrogel-Schnittstelle (31) die vom Brustkorb reflektierte Strahlung zerstreut.

5. Auf dem Brustkorb aufsitzendes Oximeter (21) gemäß einem der vorherigen Ansprüche, bei dem - bei der Anwendung - der mindestens eine Strahlungsdetektor (29) die vom Brustkorb reflektierte Strahlung erfasst, welche die Hydrogel-Schnittstelle (31) durchdringt, und die Hydrogel-Schnittstelle (31) für die Kopplung der Strahlung vom Brustkorb zum Strahlungsdetektor (29) sorgt.

6. Auf dem Brustkorb aufsitzendes Oximeter (21) gemäß einem der vorherigen Ansprüche, bei dem die Druckvorrichtung (30) eines von einer Vorspannungsvorrichtung, einer Fingerdruckvorrichtung und einem Gurt umfasst.

7. Auf dem Brustkorb aufsitzendes Oximeter (21) gemäß einem der vorherigen Ansprüche, bei dem die mindestens eine Strahlungsquelle (27) Strahlung aussendet, welche eine oder mehrere Infrarot-Spitzenwellenlängen aufweist.

8. Auf dem Brustkorb aufsitzendes Oximeter (21) gemäß Anspruch 7, welches des Weiteren eine zweite Strahlungsquelle umfasst, die Strahlung aussendet, welche eine oder mehrere Infrarot-Spitzenwellenlängen aufweist.

9. Auf dem Brustkorb aufsitzendes Oximeter (21) gemäß Anspruch 8, bei dem die mindestens eine Strahlungsquelle (27) Strahlung aussendet, welche mindestens eine erste Infrarot-Spitzenwellenlänge aufweist, und die zweite Strahlungsquelle Strahlung aussendet, welche mindestens eine zweite, unterschiedliche Infrarot-Spitzenwellenlänge aufweist.

10. Auf dem Brustkorb aufsitzendes Oximeter (21) gemäß einem der Ansprüche 7 bis 9, bei dem die oder jede Infrarot-Spitzenwellenlänge im Bereich von 600 nm bis 1500 nm liegt, beispielsweise, 780 nm, 810 nm, 820 nm, 830 nm, 840 nm, 850 nm, 870 nm, 880 nm, 890 nm, 910 nm, 940 nm, 970 nm, 1050 nm, 1070 nm, 1200 nm, 1300 nm, 1350 nm, 1450 nm, 1550 nm.

11. Auf dem Brustkorb aufsitzendes Oximeter (21) gemäß einem der Ansprüche 1 bis 6, bei dem die mindestens eine Strahlungsquelle (25) Strahlung aussendet, welche über eine oder mehrere Spitzenwellenlängen im sichtbaren Spektrum verfügt, und das Oximeter (21) des Weiteren eine zweite Strahlungsquelle umfasst, die Strahlung aussendet, welche über eine oder mehrere Spitzenwellenlängen im sichtbaren Spektrum verfügt.

12. Auf dem Brustkorb aufsitzendes Oximeter (21) gemäß einem der Ansprüche 1 bis 6, bei dem die mindestens eine Strahlungsquelle (25) Strahlung aussendet, welche über eine oder mehrere Spitzenwellenlängen im sichtbaren Spektrum verfügt, und das Oximeter des Weiteren eine zweite Strahlungsquelle umfasst, die Strahlung aussendet, welche über eine oder mehrere Infrarot-Spitzenwellenlängen verfügt.

13. Auf dem Brustkorb aufsitzendes Oximeter (21) gemäß einem der vorherigen Ansprüche, welches über ein flaches Profil verfügt.

14. Auf dem Brustkorb aufsitzendes Oximeter (21) gemäß einem der vorherigen Ansprüche, das Bestandteil eines Systems ist, welches ein oder mehrere Vitalzeichen des Patienten misst, was zum Beispiel Herzfrequenz, EKG, Atemfrequenz oder Temperatur sein können.

15. Verfahren zur Messung der Sauerstoffsättigung des Hämoglobins im Blut des Brustkorbs eines Patienten, umfassend
das Befestigen eines Oximeters (21) am Brustkorb unter Verwendung einer Druckvorrichtung (30) des Oximeters, die dazu eingerichtet ist, Druck auf das Oximeter auszuüben, um das Oximeter mit dem Brustkorb zu verbinden, wobei das Oximeter eine Hydrogel-Schnittstelle (31) umfasst,
das Betreiben von mindestens einer Strahlungsquelle (25, 27) des Oximeters, um Strahlung auf den Brustkorb auszusenden,
das Betreiben von mindestens einem Strahlungsdetektor (29) des Oximeters, um die vom Brustkorb reflektierte Strahlung zu erfassen, und
das Verwenden der durch den Detektor (29) erfassten Strahlung zur Messung der Sauerstoffsättigung des Hämoglobins im Blut des Brustkorbs.

## Revendications

1. Un oxymètre de poitrine (21) destiné à mesurer la saturation en oxygène de l'hémoglobine dans le sang de la poitrine d'un sujet, comprenant
au moins une source de rayonnement (25, 27) adaptée de façon à émettre un rayonnement sur la poitrine,
au moins un détecteur de rayonnement (29) adapté de façon à détecter un rayonnement réfléchi de la poitrine,
un dispositif de pression (30) adapté de façon à appliquer une pression à l'oxymètre de façon à relier l'oxymètre à la poitrine, et **caractérisé par** :
une interface d'hydrogel (31) située entre la au moins une source de rayonnement (25, 27) et le au moins un détecteur de rayonnement (29) et, en utilisation, la poitrine du sujet.

2. Un oxymètre de poitrine (21) selon la Revendication 1 dans lequel, en utilisation, la au moins une source de rayonnement (25, 27) émet un rayonnement au travers de l'interface d'hydrogel (31) sur la poitrine et l'interface d'hydrogel (31) diffuse le rayonnement émis à partir de la source de rayonnement (25, 27).

3. Un oxymètre de poitrine (21) selon la Revendication 1 ou 2, dans lequel, en utilisation, la au moins une source de rayonnement (25, 27) émet un rayonnement au travers de l'interface d'hydrogel (31) sur la poitrine et l'interface d'hydrogel (31) assure le couplage du rayonnement de la source (25, 27) à la poitrine.

4. Un oxymètre de poitrine (21) selon l'une quelconque des Revendications précédentes dans lequel, en utilisation, le au moins un détecteur de rayonnement (29) détecte un rayonnement réfléchi de la poitrine qui passe au travers de l'interface d'hydrogel (31), et l'interface d'hydrogel (31) diffuse le rayonnement réfléchi de la poitrine.

5. Un oxymètre de poitrine (21) selon l'une quelconque des Revendications précédentes dans lequel, en utilisation, le au moins un détecteur de rayonnement (29) détecte un rayonnement réfléchi de la poitrine qui passe au travers de l'interface d'hydrogel (31), et l'interface d'hydrogel (31) assure le couplage du rayonnement de la poitrine au détecteur de rayonnement (29).

6. Un oxymètre de poitrine (21) selon l'une quelconque des Revendications précédentes, dans lequel le dispositif de pression (30) comprend l'un quelconque des éléments parmi un dispositif de sollicitation, un dispositif à pression tactile, une ceinture.

7. Un oxymètre de poitrine (21) selon l'une quelconque des Revendications précédentes dans lequel la au moins une source de rayonnement (27) émet un rayonnement possédant une ou plusieurs longueurs d'onde de crête infrarouge.

8. Un oxymètre de poitrine (21) selon la Revendication 7 qui comprend en outre une deuxième source de rayonnement qui émet un rayonnement possédant une ou plusieurs longueurs d'onde de crête infrarouge.

9. Un oxymètre de poitrine (21) selon la Revendication 8 dans lequel la au moins une source de rayonnement (27) émet un rayonnement possédant au moins une première longueur d'onde de crête infrarouge, et la deuxième source de rayonnement émet un rayonnement possédant au moins une deuxième longueur d'onde de crête infrarouge différente.

10. Un oxymètre de poitrine (21) selon l'une quelconque des Revendications 7 à 9 dans lequel la ou chaque longueur d'onde de crête infrarouge se situe dans la plage de 600 nm à 1500 nm, par exemple 780 nm, 810 nm, 820 nm, 830 nm, 840 nm, 850 nm, 870 nm, 880 nm, 890 nm, 910 nm, 940 nm, 970 nm, 1050 nm, 1070 nm, 1200 nm, 1300 nm, 1350 nm, 1450 nm, 1550 nm.

11. Un oxymètre de poitrine (21) selon l'une quelconque des Revendications 1 à 6 dans lequel la au moins une source de rayonnement (25) émet un rayonnement possédant une ou plusieurs longueurs d'onde de crête dans le spectre visible et l'oxymètre (21) comprend en outre une deuxième source de rayonnement qui émet un rayonnement possédant une ou plusieurs longueurs d'onde de crête dans le spectre visible.

12. Un oxymètre de poitrine (21) selon l'une quelconque des Revendications 1 à 6 dans lequel la au moins une source de rayonnement (25) émet un rayonnement possédant une ou plusieurs longueurs d'onde de crête dans le spectre visible et l'oxymètre comprend en outre une deuxième source de rayonnement qui émet un rayonnement possédant une ou plusieurs longueurs d'onde de crête infrarouge.

13. Un oxymètre de poitrine (21) selon l'une quelconque des Revendications précédentes qui possède un profil mince.

14. Un oxymètre de poitrine (21) selon l'une quelconque des Revendications précédentes qui fait partie d'un système qui mesure un ou plusieurs signes vitaux du sujet, tel que l'un parmi rythme cardiaque, électrocardiogramme, rythme respiratoire, température.

15. Un procédé de mesure de la saturation en oxygène de l'hémoglobine dans le sang de la poitrine d'un sujet, comprenant la fixation d'un oxymètre (21) à la poitrine au moyen d'un dispositif de pression (30) de oxymètre adapté de façon à appliquer une pression à celle-ci de façon à relier l'oxymètre à la poitrine, l'oxymètre comprenant une interface d'hydrogel (31),
l'exploitation d'au moins une source de rayonnement (25, 27) de l'oxymètre de façon à émettre un rayonnement sur la poitrine, l'exploitation d'au moins un détecteur de rayonnement (29) de l'oxymètre de façon à détecter un rayonnement réfléchi de la poitrine, et
l'utilisation du rayonnement détecté par le détecteur (29) afin de mesurer la saturation en oxygène de l'hémoglobine dans le sang de la poitrine.
